# EUROPEAN PATENT SPECIFICATION

(11) **EP 1 709 069 B1**
(45) Date of publication and mention of the grant of the patent: **07.01.2009**
(21) Application number: 04803351.8
(22) Date of filing: 29.11.2004
(51) Int. Cl.: A61K 8/64

(54) **Epsilon-polylysine against halitosis**
Epsilon-Polylysin gegen Halitose
Epsilon-polylysine contre la mauvaise haleine

(30) Priority: 19.01.2004 BE 200400028
(43) Date of publication of application: 11.10.2006
(73) Proprietor: Huybrechts, Lucas, 2550 Kontich (BE)
(72) Inventor: Huybrechts, Lucas, 2550 Kontich (BE)
(74) Representative: Bird, Ariane
(86) International application number: PCT/EP2004/013549
(87) International publication number: WO 2005/068645

(56) References cited:
- WO-A-02/094204
- WO-A-02/103004
- JP-A- 2004 107 309
- US-A- 5 015 628
- PATENT ABSTRACTS OF JAPAN vol. 018, no. 118 (C-1172), 25 February 1994 (1994-02-25) & JP 05 310544 A (CHISSO CORP), 22 November 1993 (1993-11-22)
- REYNOLDS E C: "REMINERALIZATION OF ENAMEL SUBSURFACE LESIONS BY CASEIN PHOSPHOPEPTIDE-STABILIZED CALCIUM PHOSPHATE SOLUTIONS" JOURNAL OF DENTAL RESEARCH, INTERNATIONAL ASSOCIATION FOR DENTAL RESEARCH, US, vol. 76, no. 9, September 1997 (1997-09), pages 1587-1595, XP008028054 ISSN: 0022-0345
- ADAMSON NJ & REYNOLDS EC: "Characterization of Tryptic Casein Phosphopeptides Prepared Under Industrially Relevant Conditions" BIOTECHNOLOGY AND BIOENGINEERING, vol. 45, 1995, pages 196-204, XP002321313
- DE BOEVER E.H. ET AL: 'Assessing the contribution of anaerobic microflora of the tongue to oral malodor' JADA vol. 126, October 1995, pages 1384 - 1393

## Description

### Field of invention

The present invention relates to the products for use in the treatment of halitosis.

### Background of the invention

It is well known that caries is caused by acid which is produced by oral bacteria. Sugars, such as glucose, are an important food source for the bacteria and their availability is required for the production of acid. Streptococcus mutans (S. mutans) is considered as the most important culprit in the occurrence of caries, survives well in the oral cavity and produces relative large amounts of acid. S. mutans also produces glucosyltransferase enzymes that catalyse the production of sticky glucanes and fructanes, which are used by the bacteria for improving attachment to teeth and plaque. The saliva effectively can protect teeth partially; it contains antibacterial agents, buffering ingredients and calcium and phosphate building blocks that can be used for repair work. Notwithstanding, the protection competence of saliva is not sufficient and the tooth surface will be demolished and gradually demineralized as a result of regular acid production. Over time, holes will appear that may finally result in the loss of the teeth.

The use of fluoride for the reduction of caries has been grown enormously during the last forty years. Today flouride containing toothpastes are available worldwide. Many scientific studies have demonstrated that fluoride has the ability to (at least partially) inhibit caries; a competence based on a reduction of the demineralization process and re-enforcement of the remineralization process (US 5089255; Dental Caries, O. Fejerskow et al; Blackwell Munksgaard,2003, ISBN 1-4051-0718-9). The effect of fluoride can be enhanced with the use of co-ingredients such as zinc-components (US4396599). Notwithstanding, the protecting & inhibiting effect of fluoride is limited to approx. 25% of caries that would occur if no use would be made of fluoride. Even today the number of people in the world that are affected with caries is enormous. In addition, the toxicological profile of fluoride is under debate (especially for it's carcinogenic, inflammation, lung damaging, immunological, fluorosis and bone deformation potential). Some European countries have banned fluoride supplements from the market and the U.S. Food and Drug Administration (FDA) has limited it's use in toothpaste for home-use at 1150 ppm maximum. More recently, several strategies have been explored in the search to new protecting agents: 1. Destruction of bacteria, 2. Reduction of the production of acid, 3. Prevention of attachment of bacteria to the tooth surface and the plaque, 4. Repair of the tooth surface with calcium and phosphate building blocks and 5. the absorption of acid at the tooth surface.
1. Destruction of bacteria. Many agents with antibacterial activity against bacteria from the oral cavity, have been discovered; this includes lantibiotic peptides from bacteria (US4209508; US pending 20030118590), lytic enzymes from bacteriophages that are able to destroy the cell wall of streptococcus mutans (US 6355012, US 6399098), extracts from yeast-cells (US 4980151) or components from natural oils such as Cubeb oil or java citronella oil (US4590215: alpha cadinol). Also extracts from plants as Angelica Gigas, Akebia quinata, Camellia sinensis and Korean Ginseng Flower (J. Dent. Res. 2003; vol 82, spec. issue B, 1591 and 1670) appear to demonstrate antibiotic activity. In addition synthetic antibacterial agents have been developed that act against streptococcus mutans: N-alkyl-pyridinum chloride, bisbiguanides (Chlorohexidine, US 6143281), quaternary ammonium-galactomannans (US 4282204), polyphenols (triclosan; US 6136298) and modified histatine-peptides (US 5672351). Other strategies consist in the the stimulation of an immunological reaction against S. Mutans with peptides that have an identical structure compared to parts of the S.Mutans antigen I/II (cell wall protein; US 6024958 and US 6500433), or the use of antibodies against S.Mutans antigen I/II that have been produced with another bacteria (US 5612031). Some suggest to use bacteriophages in the fight against S.Mutans (US 4957686 and US 4891210).
2. The production of acid can be limited with the use of sugars such as erythritol (US 6238648, US6177064, US 4346116), xylitol, trehalose, palatinose (US 5985622). Despite their large-scale use in food, it is necessary to replace large amounts of glucose, and the use of an elevated dosage may result in an unwanted laxative side effect.
3. The attachment of S. Mutans to the tooth surface could possibly be limited in a variety of ways: Pyrophosphates and ZnO limits the formation of plaque and reduces the attachment possibilities (US 5455024); glucanases and dextranases degrade glucanes that are used by the bacteria to attach to plaque (US 5468479); it could be possible to use a peptide vaccin that stimulates an immunological reaction against glucosyltransferase; this enzym catalyses the production of glucanes (US 5686075); peptides with a structure similar to parts of the S. Mutans antigen I/II could compete with the antigen for attachment to plaque (US 6500433).
4. Repair work. Saliva contains calcium and phosphate ions that contribute to the repair of the tooth surface. It does also contain natural proteins (e.g. statherin) that helps preventing the precipitation of such ions, keeping them available for repair work. Casein (US 5130123) and especially also non-denaturated Casein (US 5427769) have anticariogenic competence. Caseinphoshopeptides (CPP) are made by hydrolyses of Casein, and they too have anticariogenic competence. CPP forms a complex with calcium and phosphate ions, and stimulates repair work.
5. Adhesion to the tooth surface & acid absorption.
   Adhesion allows the product to accumulate at the location where it is needed, the tooth surface. Caseinphosphopeptide as well as phosvitin, a protein found in egg, have the ability to attach; they accumulate on the surface and generate a local buffering capability that contributes to preventing the acid to dissolve the tooth (US 5.279.814 en B.Jiang et al, Biosci. Biotechnol. Biochem., 65 (5), 1187-1190,2001 en B.Jiang et al, J. Agric. Food Chem.,48, 990-994,2000 en A. Goulas et al, J. of Protein Chemistry, vol 15, no.1, 1-9,1996). Products with basic components have buffering competence. Arginine and small peptides (2-4) that contain at least one arginine amino acid, have been used to increase the pH of the plaque (US 621851, US 4225579, US 5762911 en Kanapka, Archs. oral. Biol. 28,1007-1015,1983). Also chitosan (salts) are recommended (US 4512968); but their potential is restrained by lack of solubility at neutral or basic pH. Another polyamine, polyethyleneimino fluorophosphate appears to attach to teeth and to protect against acid (US 4020019). However, it's resistance against enzymatic degradation and it's capability to attach to a cell wall, renders it irritant and toxic. The occurrence of caries is still widespread, despite the many creative research efforts that have been carried out; 99% of Germans and 94% of Italians between 35-44 years of age and 40% of Belgian children between 5-7 years of age, suffer from caries according to an epidemiological study that has been carried out in Europe a few years ago. It is therefore necessary that new products are developed with strong protection capabilities against acid attack on teeth.

JP2004107309 discloses the combined use of epsilon polylysine (or polylysine), abrasive and zinc ingredients in toothpaste-formulations for use in dental plaque control, dental calculus prevention, caries prevention, gingivitis prevention and ozostomia prevention. The Zinc component is considered to be the active antimicrobial ingredient (particularly against Streptococcus mutans). Epsilon polylysine is used as a protector for zinc against the destabilizing effect of abrasives.

De Boever et al. (1995, JADA 126:1384-1393) relates to the successful treatment of halitosis with chlorhexidine gluconate. This is indicated to be accompanied by a decrease in prevalence and number of specific putative malodorous bacteria, but not of other bacteria. More particularly D2 specifies that the anaerobic bacteria residing on the tongue play a major role in the etiology of malodor, regardless of periodontal status.

### Summary of the invention

This invention relates to the use of epsilon-polylysine as antimicrobial compound against halitosis.

Epsilon-polylysine has demonstrated antibacterial activity against a large variety of oral cavity bacteria including acid producing bacteria. It indicates it's relevance for for control of the bacterial flora in the oral cavity in general.

The products can be used in formulations to treat the oral cavity: toothpastes, gels, mouth rinses, artificial saliva's ....for patients and healthy consumers. They have an attractive toxicological profile compared to fluoride, and can be used in food systems;

Accordingly, the present invention envisages the use of epsilon-polylysine formulated as a mouth refreshing solution, a mouth rinse, mouth spray, a gel, chewing gum, candy, mints, capsules, tablets or artificial saliva as antimicrobial compound against halitosis.

### Brief description of the figures.

Fig. 1. provides a schematic representation of the reaction between epsilon polylysine and a bisphosphonylated epoxide into bisphosphonylated e-polylysine. The epoxide is made from vinylidene diphosphonate.
Fig. 2: The reaction between epsilon-polylysine and 3-hydroxy-phthalic anhydride into 3-hydroxy-phthalated epsilon-polylysine.
Fig. 3: the susceptibility of anaerobic organisms that have been isolated from the oral cavity in the presence of the products (22; bisphosphonylated epsilon-polylysine),(6; epsilon-polylysine),(10; casein phosphopeptide epsilon-polylysine copolymer) or (27; 3-hydroxy-phthalated epsilon-polylysine). The minimum inhibitory concentration (MIC) is given in mg/ml within an explored range of 0.2 to => 3.5 mg/ml.
Fig.4: The susceptibility of microaerophilic organisms that have been isolated in the oral cavity in the presence of the products (22), (6), (10)or (27) and expressed in MIC.
Fig. 5: The susceptibility of fungi (yeast alike) that have been isolated in the oral cavity in the presence of the products (22), (6), (10) or (27) and expressed in MIC.
Fig. 6: The susceptibility of aerobic organisms that have been isolated in the oral cavity and of standard strains in the presence of the products (22), (6), (10) or (27) and expressed in MIC.
Fig. 7: The susceptibility of Streptococcus spp. strains that have been isolated in the oral cavity and of a standard strain in the presence of the products (22), (6), (10) or (27) and expressed in MIC.

### Description of the invention

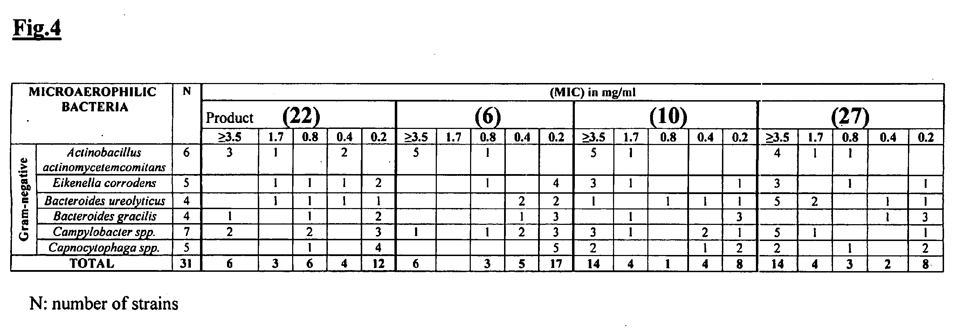

In one aspect of the invention we have demonstrated that the poly-cationic peptide, epsilon-polylysine, itself provides substantial antibacterial activity against a large variety of bacteria that reside in the oral cavity, including streptococcus mutans and anaerobic bacteria (Figure 4). It can be used as such in formulations to protect teeth and to treat the oral cavity..

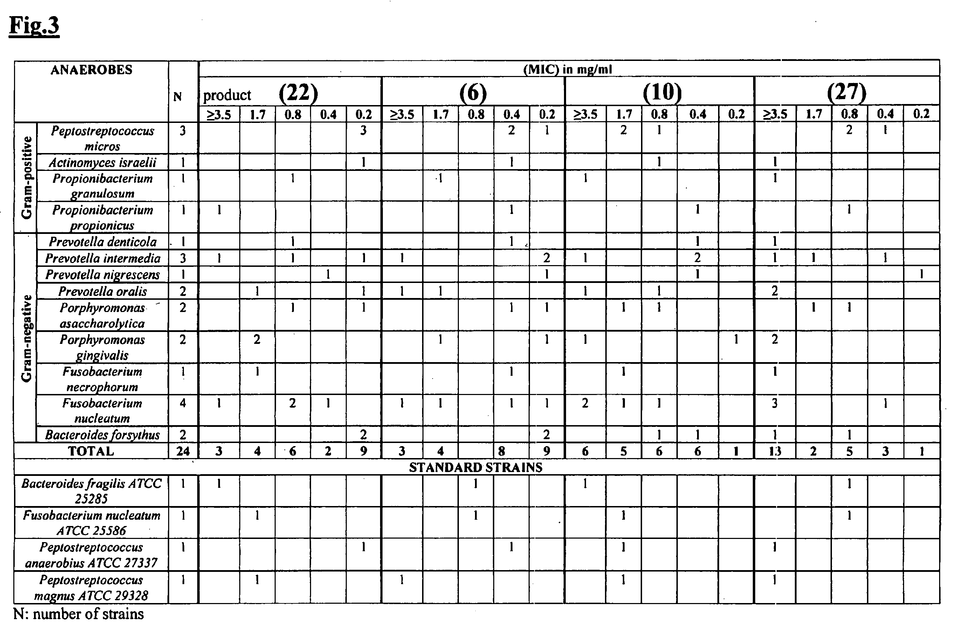

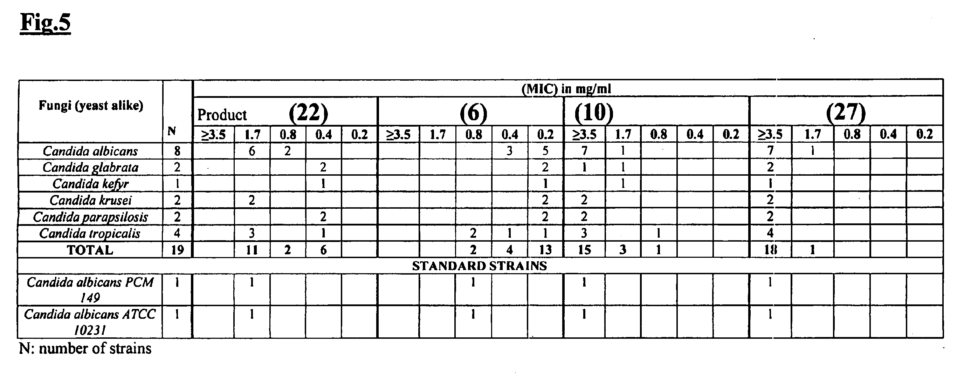

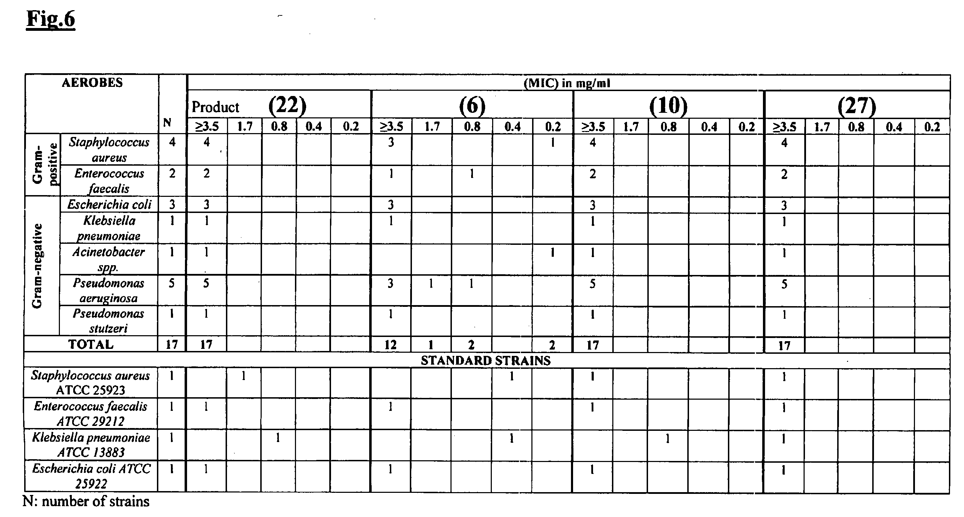

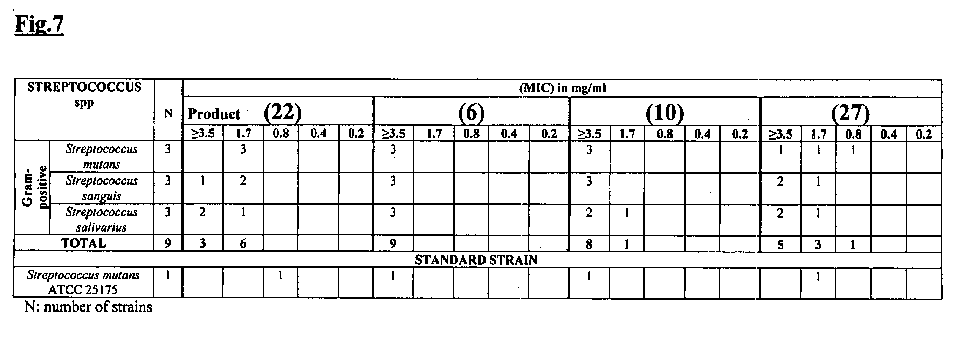

### Antibacterial activity of epsilon-polylysine and modified e-polylysine Figures 3 to 7.

In one aspect of the invention we have demonstrated that epsilon-polylysine, bisphosphonylated epsilon-polylysine, 3-hydroxy-phthalated epsilon-polylysine and the copolymer of casein phosphopeptide and epsilon-polylysine (CPP x elys)n exhibit antimicrobial activity against bacteria that are residing in the oral cavity. More than hundred different anaerobic-, aerobic-, microaerophilic bacteria and fungi, harvested all from the oral cavity, have been tested. Epsilon-polylysine exhibits the highest activity against anaerobes. From 28 anaerobic strains the growth of 24 was inhibited by concentrations in the range of 3.5-0.2 mg/ml. The gram-negative bacteria are often susceptible to a low mic-value (minimum inhibitory concentration) of =< 0.2 mg/ml, including strains of species Prevotella nigrescens, Porphyromonas asaccharolytica, Porphyromonas gingivalis, Fusobacterium nucleatum. These bacteria often take part in periodontitis, infections of dental pulp and other infections in the oral cavity. Also the gram-positive anaerobes are often susceptible to low mic-values (=< 0.2 to 0.4 mg/ml) of epsilon-polylysine. Bisphosphonylated e-polylysine demonstrated also good activity against anaerobes, but casein phophopeptide e-polylysine and 3-hydroxy-phthalated e-polylysine where less effective. Bisphosphonylated- and 3-hydroxy-phthalated e-polylysine and (CPP x elys)ₙ are not active against aerobes, but e-polylysine inhibits the growth of 7 strains among 21 in the range of 1.7 to 0.2 mg/ml. Bisphosphonylated e-polylysine inhibits 6 from 9 Streptococcus strains at 1.7 mg/ml. E-polylysine exhibits the strongest activity against microaerophilic bacteria with an inhibitory concentration of 0.2 mg/ml in the case of 17 from 31 strains. Bisphosphonylated e-polylysine too, exhibits activity (mic value of 0.2mg/ml for 12 strains from 31). Once again e-polylysine was the most active against the tested fungi with an inhibitory concentration of between 0.2 and 0.8 mg/ml for all 21 tested strains.

The antibacterial activity of e-polylysine exhibits multiple activities in the oral cavity at the same time and addresses multiple needs. It does not only contribute to the protection of teeth but also keeps the bacterial flora under control (in a similar way to what histatine, a natural peptide in saliva, does) and also helps to avoid bad mouth odor (halitosis). Indeed, many of the anaerobes that are causing infections such as parodontitis are also contributing to bad mouth odor, by producing sulfur containing compounds. It is in particular relevant to note that at the lowest concentrations (0.2 to 0.4 mg/ml) it exhibits inhibitory activity against 71 % of anaerobes that have been harvested from the oral cavity (17 from 24 species) and against 12% of aerobes harvested from the oral cavity (2 from 17). The selective antimicrobial activity of e-polylysine makes it a valuable tool for use against halitosis (bad mouth odor). E-polylysine is capable of attacking the organisms responsible for halitosis in a partially selective way. The strong antibacterial activity (especially also against fungi) makes it a good candidate for use in artificial saliva for patients with Xerostomia (dry mouth). This includes patients with oral cancer, Hodgkin's disease, Sjögren syndrome, HIV....They are often confronted with fungal infection in the oral cavity.
Epsilon-polylysine is beneficial for it's contribution to the protection of the oral cavity as a whole.

### Other ingredients in end formulations

The polymers can be used with other known ingredient in end formulations for use in the oral cavity: toothpastes, mouth refreshing solutions, mouth rinses, mouth sprays, gels, chewing gum, candies and other food systems, artificial saliva. The end formulation can contain non ionic-, anionic-, amphoteric-, cationic- or zwitterionic detergents as described in US 3988433, US 4051234, US 3959458. Non ionic detergents are condensates from hydrophilic alkylene oxide groups with hydrophobic organic components. For example: poloxamers (sold under the name Pluronic), polyoxyethylene sorbitan esters (Tween), polyethylene oxide condensates of alkyl phenols, condensates of ethylene oxide with reaction products from propylene oxide and ethylene diamine, ethylene oxide condensates from aliphatic alcohols, tertiary amine oxides with a long chain, tertiary phosphine oxide with a long chain, dialkylsulfoxides with a long chain and mixtures. Amphoteric detergents are aliphatic secondary and tertiary amines, with an aliphatic chain and with the presence of an anionic group (e.g. carboxylate, sulfonate, sulfate, phoshate, phosphonate). Anionic detergents are salts of alkylsulfates with 8 to 20 carbon atoms ( for example sodium alkyl sulfate) and salts of sulfonated monoglycerides from fatty acids with_8 to 20 carbon atoms. Examples: sodium lauryl sulfate and sodium coconut monoglyceride sulfonate, sarcosinates such as sodium lauroyl sarcosinate, sodium lauryl sulfoacetate, sodium lauroyl isethionate, sodium laureth carboxylate, sodium dodecyl benzenesulfonate or mixtures. Often the dosage of an anionic detergent is between 0.025% to 9% and preferably between 0.1% and 5% w/w. Thickeners can be used in the end formulation to provide the desired rheological profile: guar gum, carboxyvinyl polymers, carageenan, Konjac, scleroglucan, carboxymethyl cellulose, hydroxyethyl cellulose, polyoxyethylene polyoxypropylene glycol copolymers, gum karaya, gum arabic, gum tragacanth and xanthan in a concentration of 0.1% to 15%. Cross-linked polymers from acrylic acid, such as Carbopol from BF Goodrich are known in the sector. The end formulation can contain a humidifier. Polyalcohols provide a wet feeling and prevent the product from becoming hard upon contact with air. They include glycerin, sorbitol, butylene glycol, polyethylene glycol, sorbitol. The end formulation can contain abrasives: silica such as amorphous hydrated silica, sodium metaphosphate, potassium metaphosphate, tri-calcium phosphate, calcium phosphate two hydrate, calcium phosphate, calcium pyrophosphate, sodium bicarbonate, calcium bicarbonate, hydrated alumina. Sometimes are polymers used as abrasive (US 3070510): melamines, polyphenols, ureas, ureaformaldehyde...: silica bases abrasives are described in US3538230 and US3862307 (included for reference). "Syloid" (from W.R. Grace & company) and "Zeodent" (from J.M. Huber corporation) are well known in the field. Often toothpastes contain between 10% and 70% abrasive or a mixture of abrasives.

The end formulation can contain products against tooth-stone such as pyrophosphate salts such as Na.sub.4 P.sub.2 O.sub.7, K.sub.4 P.sub.2 O.sub.7, Na.sub.2 K.sub.2 P.sub.2 O.sub.7, Na.sub.2 H.sub.2 P.sub.2 O.sub.7 and K.sub.2 H.sub.2 P.sub.2 O.sub.7, sodium hexamethaphosphate, sodium tripolyphosphate and cyclic phoshphates such as sodium trimetaphosphate. The dosage is about 0.5% to 10% w/w. Anionic polycarboxylates or carboxylated chitosan could be used eventually in order to increase the anti-tooth stone effect. Copolymers of maleic anhydride with other ethylenic monomers such as methyl vinyl ether with a molecular weight between 30.000 and 1.000.000 and preferably between 30.000 and 500.000 are known under the name Gantrez (US4627977). The concentration in the end formulation is between 0.5% and 5%. Other possibilities include zinc citrate trihydrate, polyphosphates, diphosphonates (EHDP).

The end formulation can contain aroma's, often at a concentration between 0.001% and 5% and preferably between 0.5% and 1.5% w/w. Examples are: spearmint, peppermint, menthol, anethole, methyl salicylate, cassia, 1-menthyl acetate, eugenol, parsley oil, oxanone, alpha-irisone, marjoram, propenyl guaethol, vanillin, thymol, linalool, cinnamaldehyde glycerol acetal, wintergreen, sassfras clove, sage, eucalyptus, marjoram, cinnamon, lemon, lime, grapefruit, orange. The end formulation can also contain sweeteners; besides the known anticariogenic sweeteners the following products are valuable: sucrose, glucose, saccharin, dextrose, levulose, lactose, mannitol, sorbitol, fructose, maltose, xylitol, saccharin salts, thaumatin, aspartame, D-tryptophan, dihydrochalcone, acesulfame and cyclamate salts.

The end formulation can also contain ingredients against over-sensitivity (for example potassium nitrate or potassium citrate), whitening agents (hydrogen peroxide, calcium peroxide, urea peroxide), preservatives, cooling agents (carboxamides, menthol, ketals), anti-inflammatory ingredients (aspirin, ibuprofen, naproxen....).

The formulation can be provided in a one- or two-stage system. The ingredients of a "two-stage" formulation are stored seperately in two compartments and are mixed just before use.

The compositions of end formulations are known and are added for reference: for example for toothpastes US 3988433, for mouthrinses US3988433, for candies US 4083955, for chewing gum US 4083955 and for subgingival treatment US 5198220.

Toothpastes and gels often contain an abrasive (10% to 50%), a detergent (0.5% to 10%), a thickener (0.1% to 5%), a humidifier (10% to 55%), an aroma (0.04% to 2%), a sweetener (0.1% to 3%), a coloring agent (0.01% to 0.5%), water (2% to 45%) and eventually an anticariogenic product (0.05% to 10%) or a product against tartar (0.1% to 13%).

Mouth rinses and sprays often contain water (45% to 95%), ethanol (0% to 25%), a humidifier (0% to 50%), a tensio-active agent (0.01% to 7%), an aroma (0.04% to 2%), a sweetener (0.1 % to 3%) a coloring agent (0.001 % to 0.5%) and eventually an anticariogenic product (fluoride; 0.05% to 0.3%) or a product against tooth stone (0.1% to 3%).

Another formulation concerns non-abrasive gels (subgingival gels). They contain a thickener (0.1 % to 20%), a humidifier (0.1 % to 910%), an aroma (0.04% to 2%), a sweetener (0.1% to 3%), a coloring agent (0.01% to 0.5%), water (2% to 45%) and an anticariogenic or anti-tooth stone product.

Chewing gum formulations often contain gum (50% to 99%), an aroma (0.4% to 2%), a sweetener (0.01 % to 20%) and optionally an anticariogenic product. Candies, mints, capsules, tablets and other food systems have been described in US 4642903, US 4946684, US 4305502, US 4371516, US 5188825, US 5215756, US 5298261, US 3882228, US 4687662, US 4642903.

Some of the following embodiments and examples have been included for reference purposes only and do not fall under the scope of the claims.

### Examples

### A. Methods & Materials

Products. Casein phosphopeptide (CPP, Mw 1-2 kD, 1000-2000 dalton), vinylidene bisphosphonate, 3-hydroxy-phthalic anhydride, epsilon-polylysine (Mw 4100 dalton) are available on the market.

The determination of the susceptibility of microorganisms that have been isolated from the oral cavity to epsilon-polylysine, bisphosphonylated e-polylysine, casein phosphopeptide e-polylysine copolymer and 3-hydroxy-phthalated e-polylysin

The investigations included more than hundred strains isolated from the oral cavity: : anaerobes, microaerophilic bacteria, aerobes, Streptococcus spp. and yeast like fungi. The microorganisms were isolated from saliva, mucosal membrane, gingival pockets and caries decay. The susceptibility (MIC) of bacteria and yeastlike fungi were determined by means of plate dilution technique in agar. The investigated samples were dissolved in sterile distilled water (immediately before the experiment) to obtain the following concentrations: 3.5, 1.7, 0.8, 0.4 and 0.2 mg/ml and were added into appropriate agar. The plates were inoculated using a Steers multipoint inoculator. Inoculum contained 10⁶CFU/spot. In each experimental series, the growth of strains on the culture medium without the compounds investigated was checked. The concentration at which no macroscopic growth of the microbes was observed on the medium was regarded as the lowest concentration, inhibiting the growth of microbes (MIC).The examinations of the susceptibility of microbes to the peptides were carried out twice.

Anaerobes. The susceptibility of bacteria was determined by means of the agar dilution methods with Brucella agar supplemented with 5% defibrinated sheep's blood, menadione and hemin. Sample solutions with concentrations from of 3.5 - 0.2 mg/ml were added into agar. The agar plate without investigated samples was included as a bacterial growth control. The inoculum of 10⁶ CFU/ml was applied to the agar plates with Steers replicator. Incubation was performed for 48 hrs at 37°C (310°K) in anaerobic jars containing a mixture of 10% CO₂, 10% H₂ and 80% N₂, in the presence of palladium catalyst and indicator of anaerobiosis. Microaerophilic bacteria. The susceptibility of bacteria was determined by means of the agar dilution methods with Brucella agar supplemented with 5% sheep's blood. Samples solutions with concentrations from of 3.5 - 0.2 mg/ml were added into agar. The agar plate without investigated samples was included as a bacterial growth control. The inoculum of 10⁶ CFU/ml was applied to the agar plates with Steers replicator. The incubation was performed at 37°C in anaerobic jars with Campy Pak (bio Merieux) for 48 hours.

Streptococcus spp. The susceptibility of bacteria was determined by means of the agar dilution methods with Mueller-Hinton agar supplemented with 5% sheep's blood. Samples solutions with concentrations from of 3.5 - 0.2 mg/ml were added into agar. The agar plate without investigated samples was included as a bacterial growth control. The inoculum of 10⁶ CFU/ml was applied to the agar plates with Steers replicator. Incubation was performed for 24 hrs at 37°C (310°K) in anaerobic jars containing a mixture of 10% CO₂ and 90% N₂, Aerobes. The susceptibility to investigated samples was determined by means of the plate dilution technique in Mueller-Hinton agar. Samples solutions with concentrations from of 3.5 - 0.2 mg/ml were added into agar. The agar plate without investigated samples was included as a bacterial growth control. The inoculum of 10⁶ CFU/ml was applied to the agar plates with Steers replicator. Plates were incubated for 24 hrs in 37° C under aerobic conditions.

Yeast like fungi. The susceptibility of fungi to investigated samples was determined by means of the plate dilution technique in Sabouraud's agar. Samples solutions with concentrations from of 3.5 - 0.2 mg/ml were added into agar. The agar plate without investigated samples was included as a fungi growth control. The inoculum of 10⁶ CFU/ml was applied to the agar plates with Steers replicator. Plates were incubated for 24 hrs in 37° C under aerobic conditions.

### Chemical analyses & purification methods

¹H-, ¹³C-, ³¹P-NMR spectra are recorded on a Bruker AC 250 NMR. The phosphor content is determined with a Thermofinnigan Element II (HR ICPMS). Gel Permeation Chromatography is carried out with Sephadex G-25 fine gel. Ultrafiltration purification procedures are carried out with Millipore prep/scale cartridges from cellulose (cut off: 1kD) and a Millipore "Easy load masterflex" peristaltic pump.

### B. Production procedures

Casein phosphopeptide epsilon-polylysine copolymer(CPP x elys)ₙ; (10) Dissolve 10 grams of casein phosphopeptide in 86 ml deionized water and cool at 6°C with ice. Add 1.6 grams EDAC. Add 4.1 grams epsilon-polylysine after 15 minutes. Adjust the pH to 7.8 and allow the reaction to continue overnight (20 hours) at 20°C (add some additional water, 40ml, in case the viscosity is increasing too much).

Subsequently, add 300 ml deionized water and adjust the pH to 9. Ultrafiltrate with Millipore prep/scale cellulose cartridge with cut off 1000 dalton and produce 2 liter of filtrate; freeze-dry (or spray dry) the retentate.

### Bisposphonylated epsilon-polylysine (Bispho x elys) (product 18); 1-step procedure

Dissolve 165 mg vinylidene bisphosphonate (2-sodium salt; pH 6) in 1ml hydrogen peroxide (30%); add 6 mg sodium tungstate and 68 mg trifluoro-acetic acid; allow to react for one hour at room temperature. Add 120 mg epsilon-polylysine and allow to react for 5 hours at 50 to 55°C. A product is precipitating during the reaction. Remove the solvent by decantation and dissolve the precipitate in water with addition of NaOH 8N. Purify on a Sephadex 25G column with 0.01N NaOH; freeze-dry the product.

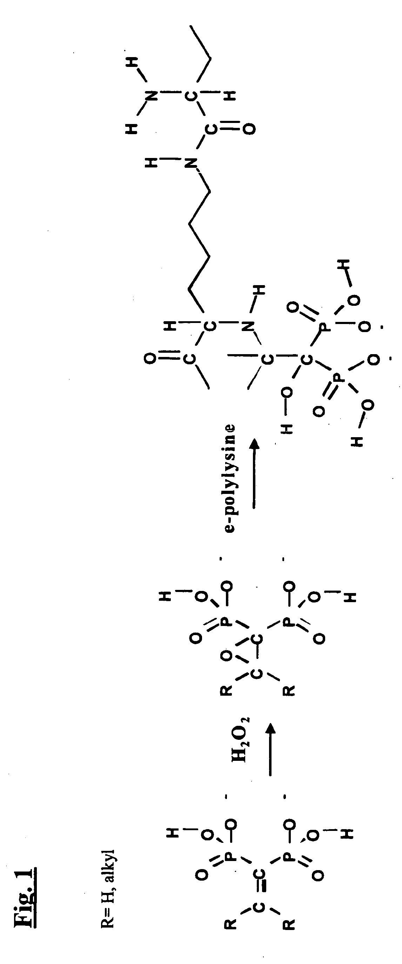

### Bisphosphonylated epsilon-polylysine Bispho x elys)(product 22) (Fig. 1); 2-step procedure (attachment of the bisphosphonate component to non-denaturated e-polylysine in isopropanol/water at acid pH).

### Step 1.

Dissolve 23 grams of vinylidene bisphosphonate (2-sodium salt, solution pH 6) in 85 ml hydrogen peroxide (30%) and add 77 mg sodium tungstate. Allow three hours to react at 60°C. Add 160 ml aceton and an 2nd layer will separate at the bottom; cool and remove the solvent by decantation. Wash the 2nd layer with aceton, remove aceton by decantation and dry.

(Remark: the reaction can also be carried out with vinylidene bisphosphona-te (4-sodium salt, solution pH is basic); removal of aceton/water can be carried out with another known method, other than decantation).

### Step 2.

Add 60 ml water to the dried product of step 1. Add 10 grams epsilon-polylysine and 1.4 ml BF3 (optionally); adjust the pH to 4 with HCl and add 67 ml isopropanol. Allow the reaction to proceed overnight at 50 to 65°C and adjust the pH afterwards to 6.9 with NaOH 8N. An 2nd layer appears with the addition of 150 ml aceton; cool and separate solvent by decantation. Wash the 2nd layer with aceton. Remove the solvent by decantation and dry. Add 300 ml water and adjust the pH to 9 with NaOH 8N. Ultrafiltrate with Millipore prepscale cartridge (cut off 1000 dalton) with a NaOH solution of 200 mg/liter. Produce 2 liter of filtrate and (freeze) dry the retentate.

¹H-NMR (D₂O): chemical shift ( integration): 1.35(2),1.55(2), 1.75(2) en 3.2(2) CHsub2 groups of epsilon-polylysine; 3.65 (1) CH-component of epsilon-polylysine (partially relocated); 3.0 (0.25): new signal, is not present in spectrum of epsilon-polylysine; CHsub2 group from coupled vinylidene component (chemical shift suggests N-CH₂ coupling and not to O-CH₂ coupling; integration suggests four bisphosphonyl groups per epsilon-polylysine molecule of (+- 4000 a 4100 dalton).

Number of bisphosphonyl groups per epsilon-polylysine molecule according to P-determination with HRICPMS: 4.3.

### Bisposphonylated epsilon-polylysine (Bispho x elys) (product 23); 2-step procedure ( attachment of the bisphosphonate component to non-denaturated e-polylysine in water at acid pH).

### Step 1.

Add 279 mg vinylidene bisphosphonate (2-sodium salt, solution pH 6) to 1ml of hydrogen peroxide (30%); add 6 mg sodium tungstate and allow the reaction to proceed for 4 hours at 70°C. Add 2 ml aceton and remove solvent from 2nd layer by decantation and dry under vacuum.

### Seep 2.

Add 120 mg epsilon-polylysine, 0.7 ml deionized water and (optionally) BF₃ to the product of step 1 and adjust the pH to 4. Allow the reaction to proceed overnight at 50°C and adjust the pH afterwards to 8.2. Purify the mixture on Sephadex 25-G column with aqueous NaOH (200 mg/L). Number of bisphosphonyl groups per epsilon-polylysine molecule according HRICPMS: 3.9.

### Bisposphonylated epsilon-polylysine (Bispho x elys); 2-step procedure (attachment of the bisphosphonate component to denaturated epsilon-polylysine in isopropanol/H2O at acid pH ).

### Step 1.

Add 279 mg vinylidene bisphosphonate (2-sodium salt, pH solution 6) to 1 ml hydrogen peroxide (30%); add 6 mg sodium tungstate and allow the reaction to proceed 4 hours at 70°C. Add 2 ml aceton and a 2nd layer appears; remove the solvent by decantation and wash the 2nd layer with aceton, remove the aceton with decantation and dry the product, the epoxide, under vacuum.

### Step 2.

Denaturate 120 mg epsilon-polylysine in 0.7 ml water in the presence of 336 mg urea over a period of 6 hours at 40°C. Add the denaturated epsilon-polylysine to the product of step 1, the epoxide; Add 1 drop of BF3 optionally and adjust the pH to 4. Add 0.8 ml isopropanol and allow the reaction to proceed overnight at 50°C and increase the pH afterwards to 6.5. Add aceton, cool and a 2nd layer appears and remove the solvent by decantation; wash the 2nd layer with aceton and remove the solvent by decantation; dry the product under vacuum. Dissolve the 1 ml product in water and purify on a Sephadex 25-G column at pH 11 (with 200 mg/L NaOH in water) and adjust pH to 6.5 afterwards and freeze-dry the product. Purify again on Sephadex G-25 column at pH 6.5. The number of bisphosphonate groups present per epsilon-polylysine molecule (HRICPMS): 11.

### Bisposphonylated epsilon-polylysine (Bispho x elys) (product 24); 2-step procedure (attachment of the bisphosphonate component to denaturated epsilon-polylysine in isopropanol/H2O at acid pH).

### Step 1.

Add 550 mg vinylidene bisphosphonate (2-sodium salt, solution pH 6) to 2ml hydrogen peroxide and add 16 mg sodium tungstate. Allow the reaction to proceed for 3 hours and 20 minutes at 65°C and at a pH of 4.6. Adjust the pH afterwards to 6.6 and add aceton. An 2nd layer appears. Cool and remove the solvent by decantation; wash the 2nd layer with aceton, remove the aceton by decantation and dry the product.

### Step 2.

Denaturate 240 mg epsilon-polylysine in 1.2 ml water in the presence of 675 mg urea over a period of 7 hours at 40°C. Add the denaturated epsilon-polylysine to the product of step 1, the epoxide, add 2 drops of BF3 optionally and adjust the pH to 4.0. Add 1.35 ml isopropanol and allow the reaction to proceed overnight at 56°C. Adjust the pH afterwards to 6.7 and add aceton. Cool and remove the solvent by decantation. Wash the remaining 2nd layer with aceton, remove the solvent by decantation and dry under vacuum. Dissolve the product in water, adjust the pH to 8.1 and purify by ultrafiltration (cut-off 1 kD; solvent: 20 mg NaOH/liter); freeze-dry the product. The number of bisphosphonate groups present per epsilon-polylysine molecule: 6.3.

### Bisposphonylated epsilon-polylysine (Bispho x elys) (product 25). 2-step procedure (attachment of the bisphosphonate component to denaturated epsilon-polylysine in water at acid pH).

### Step 1

Add 20.8 gr vinylidene diphosphonate (2-sodium salt, solution pH 6) to 42ml hydrogen peroxide (30%) and 250 mg sodium tungstate, adjust the pH to 4.6 and allow to react for 3hours at 69°C; adjust the pH afterwards to 6.4. Add 80 ml aceton (an 2nd layer appears); cool and remove the aceton/water layer by decantation; wash the 2nd layer again with aceton and remove the aceton by decantion; dry the 2nd layer under vacuum optionally.

### Step 2

Denaturate 5 gr epsilon polylysine in 9 ml water in the presence of 10.1 gr urea (4h, 20°C). Add the denaturated e-polylysine to the product from step 1 (epoxide), add 0.68 ml BF3 optionally, adjust the pH to 4 and allow the reaction to proceed overnight at 50°C. Afterwards, adjust the pH to 7.0 and ultrafiltrate with an aqueous solution of NaOH 40mg/L. Produce 2 liters of filtrate and freeze-dry the retentate.

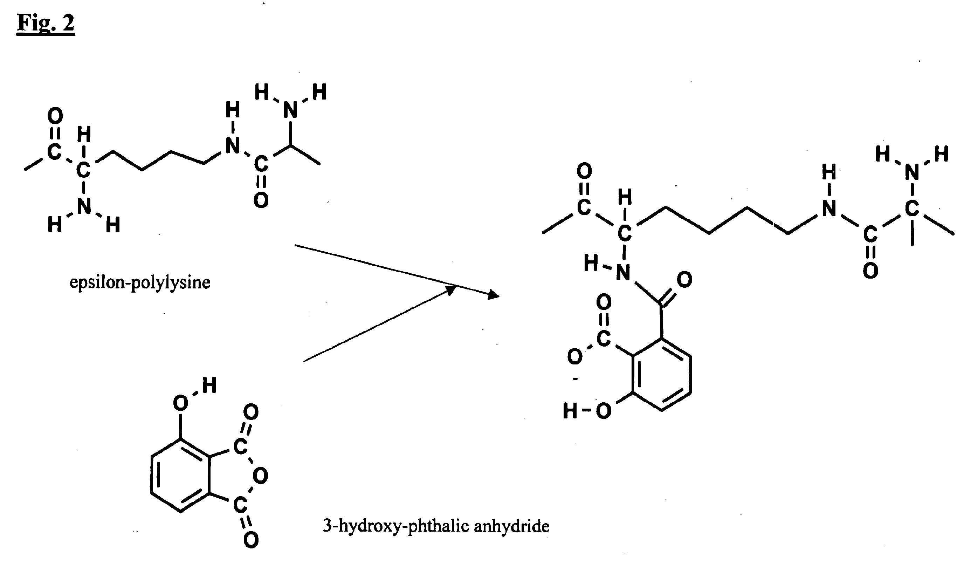

### 3-Hydroxy-phthalated epsilon-polylysine. (product 27) (Fig. 2).

Add 5.4 grams epsilon-polylysine and 3.2 grams 3-hydroxy-phthalic anhydride to 40 ml water free DMSO at 6°C. A yellow oil appears after one night at 20°C. Wash with aceton, remove the solvent by decantation and dry under vacuum. Dissolve the product in 300 ml water with the addition of NaOH untill the pH is 11. Ultrafiltrate at 35°C on a Millipore prep scale cellulose cartridge (cut off 1000 dalton) and at a pressure of 1.5 bar with 0.05N NaOH. Produce 2 liter of filtrate and freeze-dry the retentate.

## Claims

1. Use of epsilon-polylysine as antimicrobial compound against halitosis.

2. The use according to claim 1, wherein said epsilon-polylysine is formulated as a mouth refreshing solution, a mouth rinse, mouth spray, a gel, chewing gum, candy, mints, capsules, tablets or artificial saliva.

## Patentansprüche

1. Verwendung von Epsilon-Polylysin als antimikrobielle Verbindung gegen Halitose.

2. Verwendung nach Anspruch 1, wobei das Epsilon-Polylysin als eine Munderfrischungslösung, Mundspülung, als Mundspray, ein Gel, Kaugummi, Bonbons, Mints, Kapseln, Tabletten oder künstliche Speichelflüssigkeit formuliert wird.

## Revendications

1. Utilisation d'epsilon - polylysine en tant que composé antimicrobien contre la mauvaise haleine.

2. Utilisation selon la revendication 1, dans laquelle ladite epsilon - polylysine est formulée comme une solution rafraîchissante pour la bouche, une solution de rinçage pour la bouche, un vaporisateur pour la bouche, un gel, un chewing-gum, une confiserie, des bonbons à la menthe, des capsules, des comprimés ou de la salive artificielle.
